# EUROPÄISCHE PATENTSCHRIFT

(11) **EP 0 560 406 B1**
(45) Veröffentlichungstag und Bekanntmachung des Hinweises auf die Patenterteilung: **07.05.1997**
(21) Anmeldenummer: 93107242.5
(22) Anmeldetag: 01.08.1988
(51) Int. Cl.: C07D 213/75, A61K 7/00, C07D 213/73, C07D 213/74, C07D 413/14, C07D 413/12

(54) **Herstellungsverfahren für 2,5-Diamino-6-nitro-pyridinderivate sowie neue 2,5-Diamino-6-nitro-pyridinderivate**
Process for the preparation of 2,5-diamino-6-nitro-pyridine derivatives and new 2,5-diamino-6-nitro-pyridine derivatives
Procédé de préparation de dérivés de diamino-2,5-nitro-6-pyridine et nouveaux dérivés de diamino-2,5-nitro-6-pyridine

(30) Priorität: 17.08.1987 DE 3727297
(43) Veröffentlichungstag der Anmeldung: 15.09.1993
(62) Teilanmeldung aus: 88112436.6
(73) Patentinhaber: Wella Aktiengesellschaft, 64295 Darmstadt (DE)
(72) Erfinder: Clausen, Thomas, Dr., W-6146 Alsbach (DE)

(56) Entgegenhaltungen:
- DE-A- 1 949 750
- DE-A- 3 334 030
- CHEMICAL ABSTRACTS, vol. 95, 1981, Columbus, Ohio, US; abstract no. 24817j, URAL POLYTECHNIC INSTITUTE Seite 675 ; & SU-A-773 043
- CHEMICAL ABSTRACTS, vol. 90, 1979, Columbus, Ohio, US; abstract no. 152079d, G. A. MOKRUSHINA ET AL. 'Direct amination in the pyridine series' Seite 605 ; & KHIM. GETEROTSIKL. SOEDIN. Nr. 1, 1979, Seite 131

## Beschreibung

Die Erfindung betrifft ein Verfahren zur Herstellung von 2,5-Diamino-6-nitro-pyridinen sowie neue 2,5-Diamino-6-nitro-pyridine.

Die 2,5-Diamino-6-nitro-pyridine stellen einerseits interessante Zwischenprodukte für die Synthese von Arzneimitteln und Farbstoffen dar, andererseits sind sie als nichttoxische Nitrofarbstoffe in Haarfärbemitteln interessant. In der Literatur (vergleiche zum Beispiel J. C. Johnson, Hair dyes, Noyes Data Corp., Park Ridge, New Jersey, USA 1973, Seiten 43-91 und Seiten 113-124) werden Nitrofarbstoffe ausführlich beschrieben. Gelbe, rote, violette und blaue Farbstoffe lassen sich bislang jedoch nur durch unterschiedlich subsituierte 2-Nitro-p-phenylendiamine herstellen. Da nun in letzter Zeit Zweifel an der physiologischen Unbedenklichkeit mancher 2-Nitro-p-phenylendiaminderivate aufgekommen sind, besteht ein großer Bedarf an Farbstoffen mit einem nichtbenzoiden Grundkörper, wie zum Beispiel Pyridinderivaten, welche bei möglichst gleicher Farb-Bandbreite bessere physiologische Eigenschaften aufweisen.

Die 2,5-Diamino-6-nitro-pyridine lassen sich nicht auf dem aus der Benzolchemie bekannten Weg über eine Nitrierung des 2,5-Diamino-pyridins herstellen, da dieses bei der Nitrierung oxidiert wird. Zudem wäre beim Gelingen einer solchen Nitrierung zu erwarten, daß die Nitrogruppe in 3-Stellung eintritt.

Das einzige bekannte Herstellungsverfahren für 2,5-Diamino-6-nitro-pyridinderivate ist in der russischen Patentschrift 773 049 sowie in den Veröffentlichungen der gleichen Autoren in Khim. Geterotsikl. Soedin 1979 (1), Seite 131 und 1981 (5), Seite 654 ff. beschrieben. Voraussetzung ist dort jedoch die Herstellung des 3-Azido-2-nitro-pyridins(VI), das dann mit Aminen umgesetzt werden kann: 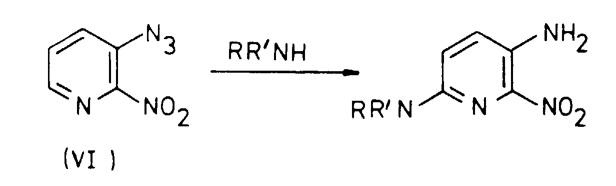 Da das Azid (VI) thermolabil ist, kann das vorstehende Herstellungsverfahren nicht in großtechnischem Maßstab angewandt werden.

Es bestand daher die Aufgabe, ein Herstellungsverfahren für 2,5-Diamino-6-nitro-pyridine zur Verfügung zu stellen, welches ausgehend von preiswerten und in technischen Mengen verfügbaren Verbindungen die Synthese einer Vielzahl von 2,5-Diamino-6-nitro-pyridinderivaten ermöglicht.

Gegenstand der vorliegenden Erfindung ist daher ein Verfahren zur Herstellung von 2,5-Diamino-6-nitro-pyridinderivaten der allgemeinen Formel (I) 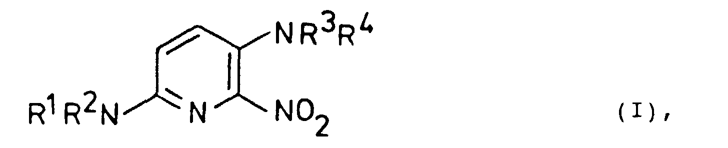 worin R¹ bis R⁴ unabhängig voneinander Wasserstoff oder einen der Reste Alkyl, Hydroxyalkyl, Dihydroxyalkyl, Alkoxyalkyl, Alkoxycarbonyl, hydroxy- oder chlorsubstituiertes Alkoxycarbonyl, Aminoalkyl sowie mit Alkyl-oder Hydroxylgruppen substituiertes Aminoalkyl bedeuten, wobei die Alkylreste jeweils 1 bis 4 Kohlenstoffatome aufweisen oder durch R¹ und R² beziehungsweise R³ und R⁴ ein Oxazolidinonring gebildet wird, dadurch gekennzeichnet, daß ein 2,5-Diamino-pyridinderivat der allgemeinen Formel (II) 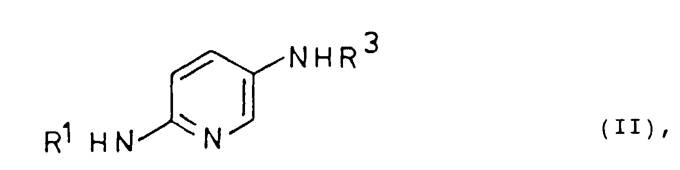 worin R¹ und R³ die vorstehend angegebene Bedeutung haben, mit einem Chlorameisensäurealkylester zu dem N²,N⁵-Bis-alkoxycarbonyl-2,5-diamino-pyridinderivat der allgemeinen Formel (III) 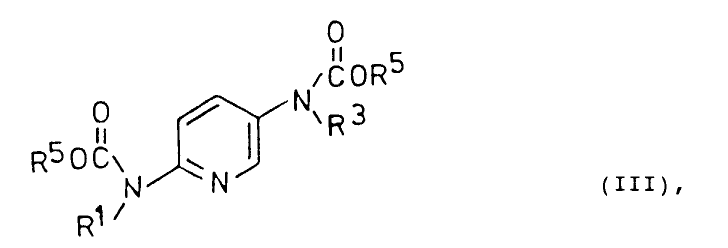 worin R¹ und R³ die vorstehend angegebene Bedeutung haben und R⁵ einen Alkyl- oder Chloralkylrest mit jeweils 1 bis 4 Kohlenstoffatomen darstellt, umgesetzt wird, dieses sodann in 6-Stellung zu dem N²,N⁵-Bis-al-koxycarbonyl-2,5-diamino-6-nitro-pyridinderivat der allgemeinen Formel (IV) 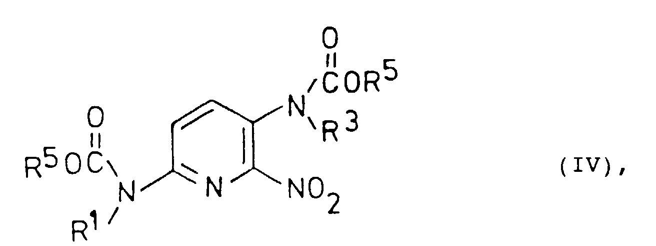 worin R¹, R³ und R⁵ die vorstehend angegebene Bedeutung haben, nitriert wird und anschließend, falls die Verbindung der Formel (I) keine Alkoxycarbonylreste aufweist, das Produkt (IV) durch Abspaltung der Alkoxycarbonylreste und durch darauffolgende, für die Alkylierung von aromatischen Aminen bekannte Alkylierungsreaktionen mit Alkylierungsmitteln, durch welche die vorstehend für R¹ bis R⁴ angegebenen Reste eingeführt werden können, beispielsweise Alkylhalogeniden, in das 2,5-Diamino-pyridinderivat der allgemeinen Formel (I) übergeführt wird.

Die Tatsache, das N²,N⁵-Bis-alkoxycarbonylderivate des 2,5-Diaminopyridins ohne das Auftreten von Oxidationsprodukten nitriert werden können, wobei die Nitrogruppe in 6-Stellung eintritt, obgleich die normalerweise elektronenreichere Position 3 nicht durch einen Substituenten besetzt ist, ist völlig überraschend.

Besonders vorteilhafte Schutzgruppen sind die Ethoxycarbonylgruppe, die preiswert und leicht einführbar ist, sowie die 2-Chlorethoxycarbonylgruppe, die sich nach der Nitrierung in eine 2-Hydroxyethylgruppe überführen läßt und damit Abspaltung der Schutzgruppe und Alkylierung der Aminogruppe in einem Reaktionsschritt vereinigt, wodurch insbesondere die Herstellung der 2-Hydroxyethylderivate außerordentlich erleichtert wird.

Das neue erfindungsgemäße Verfahren geht von dem Preiswerten und in technischen Mengen verfügbaren 2,5-Diamino-pyridin oder dessen Derivaten aus, die in einer Stufe zum Bis-Carbamat umgesetzt werden können. Selbstverständlich lassen sich die Bis-Carbamate auch durch Umlagerungsreaktionen aus den preiswerten Pyridin-2,5-dicarbonsäurederivaten gewinnen, wie zum Beispiel bei H. Meyer und F. Staffen, Monatsh. Chem. 34, 517 ff. (1913), beschrieben.

Besonders geeignet ist das neue Verfahren für solche Verbindungen der Formel (I), worin die Reste R¹ bis R⁴ unabhängig voneinander Wasserstoff, Methyl, Ethyl oder 2-Hydroxyethyl bedeuten oder durch R¹ und R² und/oder R³ und R⁴ ein Oxazolidinonring gebildet wird.

So wird durch das neue Verfahren beispielsweise die Herstellung von 2,5-Diamino-6-nitro-pyridin ((I); R¹ bis R⁴ = Wasserstoff) gegenüber dem aus der russichen Patentschrift 773 049 bekannten Verfahren erheblich erleichtert.

Ebenfalls leicht lassen sich durch das erfindungsgemäße Verfahren 2,5-Diamino-6-nitro-pyridinderivate darstellen, in denen R¹ die vorstehend angegebene Bedeutung hat, jedoch nicht Wasserstoff ist, und die Reste R², R³ und R⁴ Wasserstoff darstellen. Die als Ausgangsprodukte verwendeten, am Aminstickstoff in 2-Stellung substituierten 2,5-Diamino-6-nitro-pyridinderivate lassen sich, wie das nachfolgende Schema zeigt, vorteilhaft durch nucleophilen Austausch des Chloratoms im 2-Chlor-5-nitro-pyridin gewinnen:

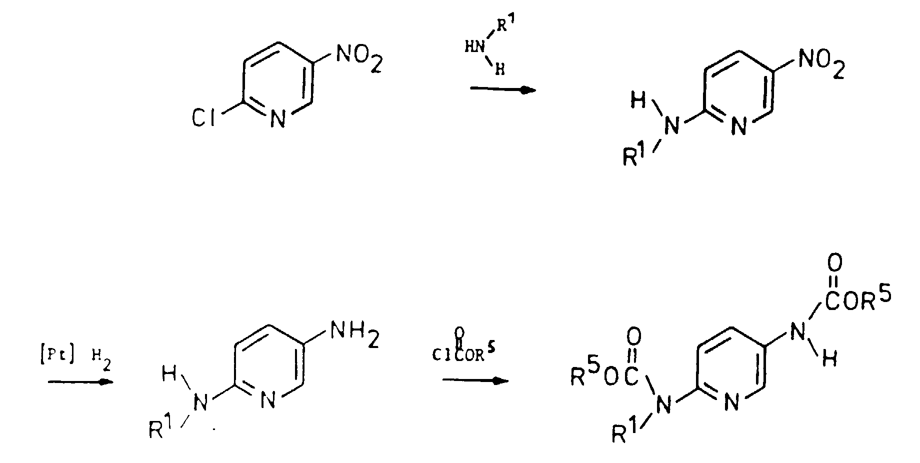

In einer speziellen Ausführungsform des neuen Verfahrens wird die Herstellung von hydroxyalkylierten 2,5-Diamino-6-nitro-pyridinen, beispielsweise 2,5-Bis((2'-hydroxyethyl)amino)-6-nitro-pyridin, ermöglicht, wobei 2,5-Diamino-pyridin zunächst mit Chlorameisensäurechlorethylester zum N²,N⁵-Bis-(2'-chlorethoxycarbonyl)-2,5-diamino-pyridin umgesetzt wird, das Produkt sodann in 6-Stellung nitriert wird und anschließend die 2-Chlorethoxycarbonylreste durch Umsetzung mit Ammoniak oder Alkylaminen, beispielsweise Ethylamin, in Oxazolidinonreste umgewandelt werden, welche danach durch Einwirkung von ethanolischer Alkalihydroxidlösung zu 2-Hydroxyethylresten umgesetzt werden:

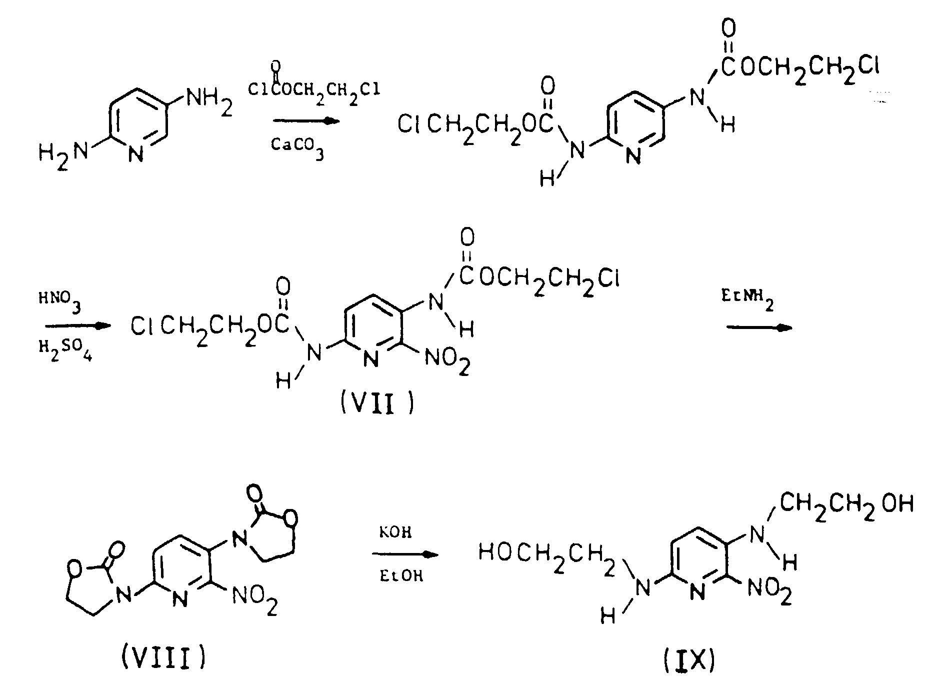

Das intermediär gebildete Bis-Oxazolidinon (VIII) kann isoliert werden, die Reaktion von (VII) zu (IX) läßt sich aber auch in einem Schritt durchführen. Die Herstellung verschiedener Derivate wird in den Beispielen beschrieben.

Durch das neue Herstellungsverfahren wird eine ganze Reihe von 2,5-Diamino-6-nitro-pyridinderivaten, wie zum Beispiel die 2,5-Bis-carbamate, die 2,5-Bis-alkyl-aminoderivate oder durch Alkylierung die Trialkyl-2,5-diamine, neu erschlossen.

Gegenstand der Erfindung sind weiterhin neue 2,5-Diamino-6-nitro-pyridinderivate der allgemeinen Formel (V) 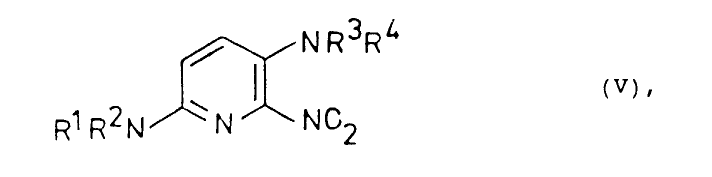 in der R¹ bis R⁴ die vorstehend angegebene Bedeutung haben, mit der Maßgabe, daß R¹ bis R⁴ nicht ausschließlich Wasserstoff bedeuten und daß, wenn R¹ und R² Alkyl bedeuten, R³ und R⁴ von Wasserstoff verschieden sind.

Beispiele für neue 2,5-Diamino-6-nitro-pyridinderivate der allgemeinen Formel (V) sind
2,5-Bis((2'-hydroxyethyl)amino)--6-nitro-pyridin, 2-Ethyl-amino-5-((2'-hydroxyethyl)amino)-6-nitro-pyridin, 2,5-(Bis((2'-hydroxyethyl)amino)-2-N-ethyl-6-nitro-pyridin, 5-Amino-2-((2'-hydroxyethyl)amino)-6-nitro-pyridin und 2,5-Bis(1',3'-oxazolidin-3'-yl-2'-on)-6-nitro-pyridin.

Die erfindungsgemäßen neuen Verbindungen der allgemeinen Formel (V) stellen beispielsweise für die Färbung von menschlichen Haaren hervorragend geeignete, gelborange bis blauviolette, direkt auf das Haar aufziehende Nitrofarbstoffe sowie interessante Zwischenprodukte für die Synthese von Arzneimitteln dar.

Die nachfolgenden Beispiele sollen die Erfindung näher erläutern, ohne sie darauf zu beschränken.

### Herstellungsbeispiele

### Beispiel 1: 2,5-Bis(ethoxycarbonylamino)-6-nitropyridin

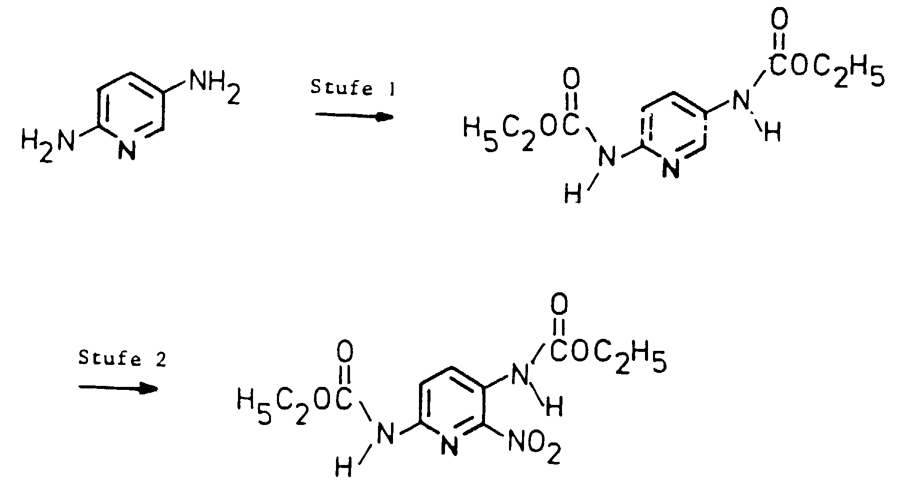

### Stufe 1: 2,5-Bis(ethylcarbonylamino)-pyridin

10 g 2,5-Diaminopyridin werden zusammen mit 20 g Calciumcarbonat unter Rühren in 100 ml Dioxan aufgeschlämmt und auf 70 Grad Celsius erwärmt. Man läßt 20 ml Chlorameisensäureethylester langsam zutropfen und rührt über Nacht bei 70 Grad Celsius. Anschließend wird unter Nachwaschen mit Dioxan noch heiß vom überschüssigen Calciumcarbonat abgesaugt und das Filtrat im Vakuum eingeengt. Der Rückstand kristallisiert aus ethanolischer Lösung aus. Die Verbindung läßt sich auch nach H. Meyer und F. Staffen, Monatsh. Chemie 34, Seite 517 ff. (1913) darstellen.

### Stufe 2: 2,5-Bis(ethoxycarbonylamino)-6-nitro-pyridin

40 ml konzentrierte Schwefelsäure werden auf 8 Grad Celsius gekühlt, und unter Rühren werden 5 g der Verbindung der Stufe 1 eingetragen. Bei 5 Grad Celsius werden dann tropfenweise 5 ml konzentrierte Salpetersäure zugesetzt und noch 1 Stunde lang gerührt. Anschließend wird der Ansatz auf 300-400 g Eis gegossen, und die ausgefallenen Kristalle werden durch Absaugen isoliert. Man erhält 4 g einer gelben kristallinen Verbindung, die nach der Umkristallisation aus Ethanol bei 193 Grad Celsius schmilzt.
¹H-NMR-Spektrum.
10,38 (s, NH), 9,72 (s,NH), 8,23 (d, J = 9 Hz, 4-H), 8,10 (d, J = 9 Hz, 3-H), 4,19 (q, J = 7 Hz, OCH₂), 4,13 (q, J = 7 Hz, OCH₂), 1,25 (t, J = 7 Hz, CH₃), 1,23 (t, J = 7 Hz, CH₃).

Für dieses und alle anderen NMR-Spekturen gilt:
Alle Angaben in delta (ppm) bezogen auf Tetramethylsilan als Standard mit delta = 0. Als Lösungsmittel diente,
soweit nicht anders angegeben, DMSO-d₆.
s = Singulett, d = Dublett, t = Triplett
q = Quartett, m = Multiplett.
Massenspektrum:
298 (M⁺, 65), 253 (11), 252 (32), 225 (15), 224 (100), 180 (20), 178 (36), 154 (12), 152 (39), 134 (18), 108 (35), 107 (48), 106 (22), 96 (10), 95 (15), 81 (42), 80 (50), 79 (92), 54 (18), 53 (21), 52 (14).

Für dieses und alle anderen Massenspektren gilt:
Alle Angaben in m/e (relative Intensität) für alle Peaks, welche eine relative Intensität von größer oder gleich 10 Prozent aufweisen.

### Beispiel 2: 2,5-Diamino-6-nitro-pyridin

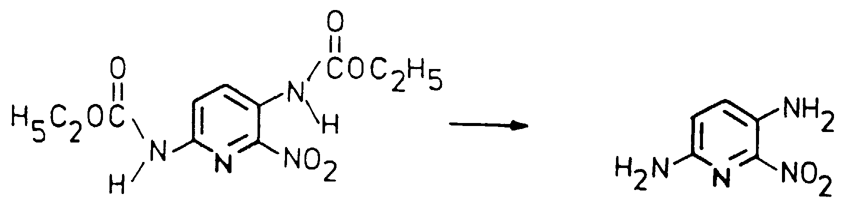

4,0 g des Biscarbamats des Beispiels 1 werden in 200 ml ethanolischer 3-prozentiger Kaliumhydroxidlösung 18 Stunden lang unter Rückfluß gekocht. Anschließend wird filtriert, das Filtrat mit konzentrierter Schwefelsäure angesäuert und im Vakuum eingeengt. Der Rückstand wird in Methanol aufgenommen, worauf das gesuchte Produkt als Hydrochlorid auskristallisiert (Schmelzpunkt 241 Grad Celsius).
¹H-NMR-Spektrum:
7,84 (s, breit; NH₂; Signal verschwindet beim Schütteln der Probe mit D₂O), 7,74 (d, J = 10 Hz; 4-H), 7,26 (d, J = 10 Hz; 3-H).
Massenspektrum:
154 (M⁺, 58), 108 (47), 80 (57), 79 (20), 78 (13), 54 (72), 53 (32), 52 (20), 37 (61), 35 (100).

### Beispiel 3: N-(5'-Ethoxycarbonylamino-6'-nitro-pyrid-2'-yl)-N-ethoxycarbonyl-2-aminoethyl-ethyl carbonat

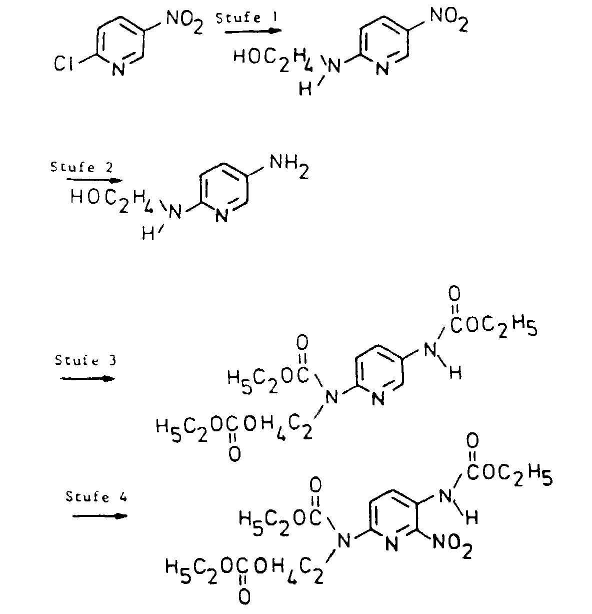

### Stufe 1: 2-((2'-Hydroxyethyl)amino)-5-nitro-pyridin

10 g 2-Chlor-5-nitro-pyridin werden in 30 ml Ethanolamin bei Raumtemperatur gerührt. Nach etwa 5 Minuten beginnt eine exotherme Reaktion, die nach 1 Stunde beendet ist, der Ansatz wird auf 200 g Eis gegossen und der gelbe Niederschlag durch Absaugen isoliert. Nach dem Trocknen erhält man 9,5 g der Aminoverbindung.

### Stufe 2: 5-Amino-2-((2'-hydroxyethyl)amino)-pyridin

9,5 g des Produkts aus Stufe 1 werden in 200 ml Methanol an Platin mit Wasserstoff bei Normaldruck und Raumtemperatur katalytisch reduziert. Nach der Aufnahme von 3,7 Liter Wasserstoff wird vom Katalysator abfiltriert und das Filtrat im Vakuum eingeengt. Der Rückstand wird durch Luftsauerstoff leicht oxidiert und daher ohne Reinigung in Stufe 3 eingesetzt. Die Diaminoverbindung ist zwar in Form des Dihydrochlorids im Handel erhältlich, jedoch ist Voraussetzung für das Gelingen der Stufe 3 die Verwendung der freien Base, die sich auf dem beschrieben Weg leicht in guter Reinheit herstellen läßt.

### Stufe 3: N-(5'-Ethoxycarbonylamino-pyrid-2'-yl)-N-ethoxycarbonyl-2-aminoethyl-ethylcarbonat

Die Diaminoverbindung der Stufe 2 wird in 120 ml Dioxan gelöst. Nach Zusatz von 12 g Calciumcarbonat wird unter Rühren auf 80 Grad Celsius erwärmt und man läßt 10 ml Chlorameisensäureethylester langsam zutropfen. Nach 18 stündigem Rühren bei 80 Grad Celsius wird filtriert, das Filtrat im Vakuum eingeengt und das erhaltene dunkle Öl ohne weitere Reinigung in Stufe 4 eingesetzt (Ausbeute 5,0 g).

### Stufe 4: N-(5'-Ethoxycarbonylamino-6'-nitro-pyrid-2'-yl)-N-ethoxycarbonyl-2-aminoethyl-ethylcarbonat

Die Nitrierung wird analog Beispiel 1, Stufe 2 durchgeführt. Aus 5 g der Vorstufe werden 2,4 g der Nitroverbindung erhalten.
¹H-NMR-Spektrum:
9,83 (s, NH; Signal verschwindet beim Schütteln der Probe mit D₂O), 8,20 (d, J = 9 Hz, 4-H), 8,13 (d, J = 9 Hz, 3-H), 4,4 - 3,9 (m aus 5 einzelnen q: OCH₂- bzw. N-CH₂-), 1,27 (t, J = 7 Hz, CH₃), 1,24 (t, J = 7 Hz, CH₃), 1,15 (t, J = 7 Hz, CH₃).
Massenspektrum:
414 (M⁺, 17), 308 (16), 307 (100), 252 (10), 239 (16), 235 (14), 178 (21), 167 (11), 164 (17), 120 (12), 89 (20), 45 (11).

### Beispiel 4: 5-Amino-2-((2'-hydroxyethyl)amino)-6-nitro-pyridin

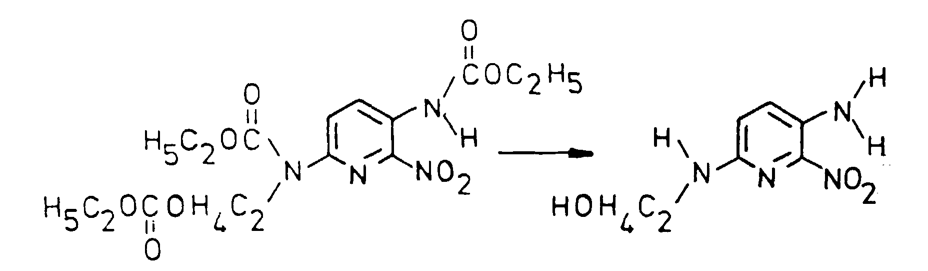

5,0 g des Tris-carbamats aus Beispiel 3 werden 2 1/2 Tage lang bei Raumtemperatur in 100 ml 3-prozentiger ethanolischer Kaliumhydroxidlösung gerührt, nach dieser Zeit filtriert und nochmals 2 Tage lang mit weiteren 100 ml der Kaliumhydroxidlösung gerührt. Anschließend wird wiederum filtriert, die Filtrate werden vereinigt und mit Essigsäure auf einen pH-Wert von 5 bis 6 eingestellt. Sodann wird der Alkohol im Vakuum abdestilliert und der Rückstand aus Ethanol umkristallisiert. Man erhält 1,3 g rote Kristalle mit einem Schmelzpunkt von 185 Grad Celsius.
Massenspektrum:
198 (M⁺, 29), 168 (10), 167 (94), 154 (14), 151 (11), 137 (12), 121 (24), 120 (72), 108 (13), 107 (21),106 (12), 96 (11), 95 (10), 94 (31), 93 (62), 92 (29), 81 (48), 80 (62), 79 (91), 67 (33), 66 (41), 65 (17), 64 (24), 54 (100), 53 (82), 51 (75).

### Beispiel 5: 2,5-Bis(2'-chlorethoxycarbonylamino)-6-nitropyridin

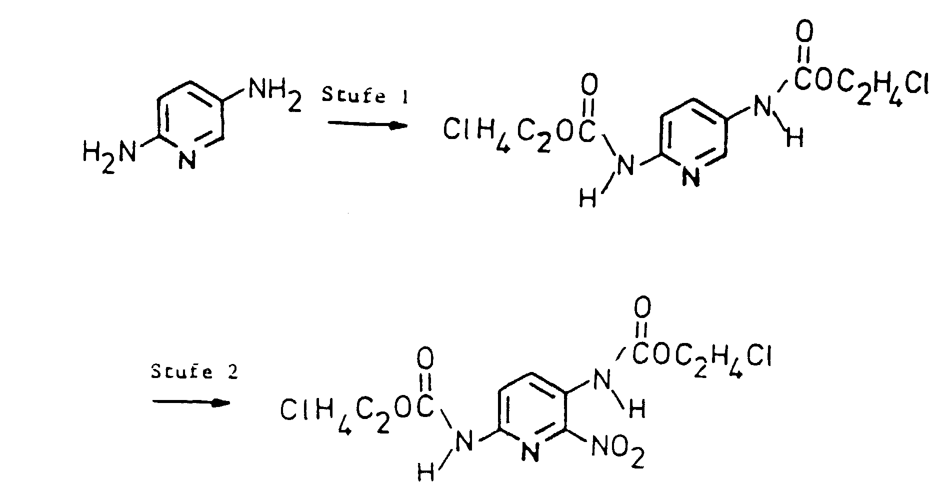

### Stufe 1: 2,5-Bis(2'-chlorethoxycarbonylamino)-pyridin

Wie in Beispiel 1, Stufe 1 beschrieben, werden 30 g 2,5-Diaminopyridin in 200 ml Dioxan unter Zusatz von 60 g Calciumcarbonat mit 40 ml Chlorameisensäurechlorethylester umgesetzt. Man erhält nach der Umkristallisation 22 g farblose Kristalle vom Schmelzpunkt 198 Grad Celsius.

### Stufe 2: 2,5-Bis(2'-chlorethoxycarbonylamino)-6-nitro-pyridin

Die Nitrierung analog Beispiel 1, Stufe 2 (Ansatz: 22 g Vorstufe der Stufe 1, 200 ml konzentrierte Schwefelsäure, 20 ml konzentrierte Salpetersäure, 800 g Eis) ergibt 18 g des gewünschten Produkts in Form farbloser Kristalle vom Schmelzpunkt 118 bis 119 Grad Celsius.
Massenspektrum:
320 (2), 318 (10), 316 (14; m/e 320, 318 und 316: M⁺), 322 (19), 320 (29), 178 (15), 80 (10), 79 (31), 65 (30), 63 (100).

### Beispiel 6: 2,5-Bis(1',3'-oxazolidin-3'-yl-2'-on)-6-nitro-pyridin

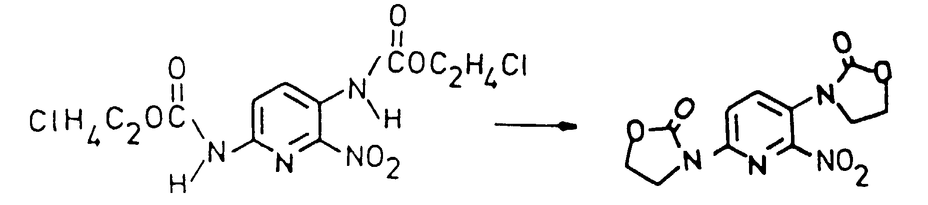

5 g des Bis-Carbamats des Beispiels 1 werden in 20 ml einer mit NH₃-Gas gesättigten Methanol lösung in einem Autoklaven 3-Stunden lang auf 120 Grad Celsius erhitzt. Nach Abkühlen und Einengen kristallisieren 4,0 g des Bis-Oxazolidinons vom Schmelzpunkt 221 Grad Celsius.
¹H-NMR-Spektrum:
8,46 (d, J = 9 Hz, 4-H), 8,29 (d, J = 9 Hz, 3-H), 4,65-4,4 (m, 0-CH₂-), 4,27 - 4,02 (m, N-CH₂).
Massenspektrum:
294 (15, M⁺), 249 (12), 248 (100), 132 (15), 118 (10), 117 (21), 106 (10), 105 (33), 92 (15), 79 (29), 78 (12), 64 (22), 52 (16).

### Beispiel 7: 2,5-Bis-((2'-hydroxyethyl)amino)-6-nitro-pyridin

5 g des Bis-oxazolidinons des Beispiels 6 werden bei Raumtemperatur 4 Tage lang in 100 ml 3-prozentiger ethanolischer Kaliumhydroxidlösung gerührt. Anschließend wird vom Niederschlag abgesaugt und das Filtrat mit Essigsäure auf einen pH-Wert von 5 bis 6 eingestellt. Das Ethanol wird im Vakuum verdampft und der Rückstand in 50 ml Wasser aufgenommen. Diese Lösung wird wiederholt mit Essigester extrahiert, die vereinigten Extrakte über Natriumsulfat getrocknet und bis zur beginnenden Kristallisation eingeengt. Man erhält 2,0 g des Produkts in Form grüner Kristalle vom Schmelzpunkt 158 Grad Celsius.

| Elementaranalyse | | |
|---|---|---|
| | berechnet | gefunden |
| % C | 44,65 | 44,67 |
| % H | 5,78 | 5,65 |
| % N | 23,13 | 22,98 |

¹H-NMR-Spektrum:
7,99 (t, J = 5 Hz, OH; das Signal verschwindet beim Schütteln der Probe mit D₂O), 7,50 (d, J = 10 Hz, 4-H), 7,02 (d, J = 10 Hz, 3-H), 6,58 (t, J = 5 Hz, OH; das Signal verschwindet beim Schütteln der Probe mit D₂O); 5,05 - 4,6 (breit, NH, das Signal verschwindet beim Schütteln der Probe mit D₂O), 3,55 (t, J = 5 Hz, breite Signale; O-CH₂-; nach Schütteln der Probe mit D₂O schärfere Absorption), 3,45-3,20 (m, breite Signale, N-CH₂).
Massenspektrum:
248 (52, M⁺), 211 (100), 164 (10), 150 (21), 136 (14), 122 (14), 121 (24), 120 (20), 119 (13), 108 (13), 107 (15), 106 (23), 105 (15), 94 (18), 93 (38), 92 (15), 80 (19), 79 (32), 78 (15), 66 (25), 64 (15), 54 (19), 53 (15), 52 (23).

### Beispiel 8: 2,5-Bis(2'-chlorethoxycarbonylamino)-2-N-ethyl-6-nitro-pyridin

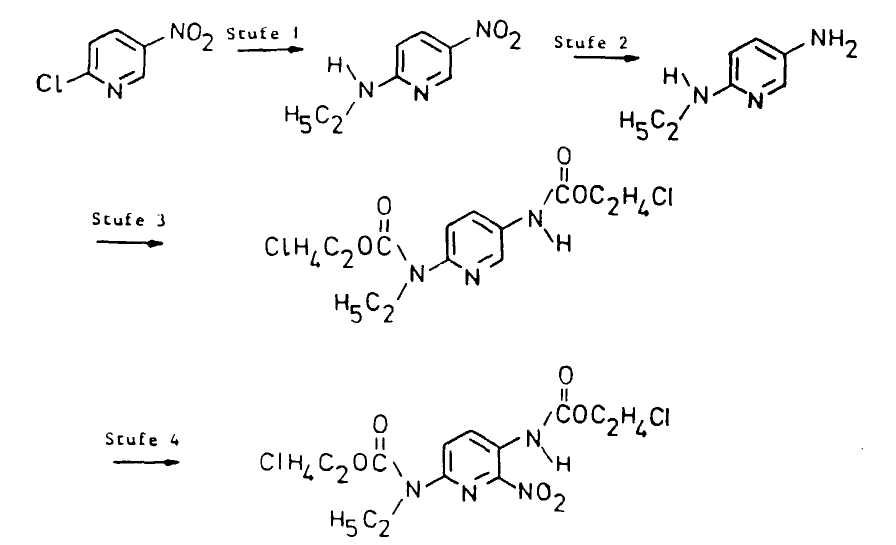

### Stufe 1: 2-Ethylamino-5-nitro-pyridin

10 g 2-Chlor-5-nitro-pyridin werden mit 30 ml Ethylamin versetzt; nach wenigen Minuten setzt eine exotherme Reaktion ein, nach deren Abklingen noch 30 Minuten lang gerührt wird. Der Ansatz wird auf 150 g Eis gegossen und von den ausgefallenen Kristallen abgesaugt. Nach der Umkristallisation aus Ethanol erhält man 9,6 g gelbe Kristalle vom Schmelzpunkt 118 Grad Celsius.
¹H-NMR-Spektrum (in CDCl₃):
9,01 (d, J = 3 Hz, 6-H), 8,18 (dd, J₁ = 3 Hz, J₂ = 10 Hz, 4-H), 6,37 (d, J₂ = 10 Hz, 3-H), 5,5 (breit, NH), 3,46 (dq, J₁ = 6 Hz, NH-Kopplung; J₂ = 7 Hz, CH₂), 1,31 (t, J = 7 Hz, -CH₃).

### Stufe 2: 5-Amino-2-ethylamino-pyridin

9 g der Nitroverbindung der Stufe 1 werden in Methanol an Platin mit Wasserstoff bei Normaldruck und Raumtemperatur hydriert. Nach Aufnahme der theoretischen Menge Wasserstoff wird vom Katalysator abfiltriert, eingeengt und der Rückstand (6,8 g) ohne weitere Reinigung in Stufe 3 eingesetzt.

### Stufe 3: 2,5-Bis(2'-chlorethoxycarbonylamino)-2-N-ethyl-pyridin

6,8 g der Diaminoverbindung der Stufe 2 werden in 30 ml Dioxan gelöst und mit 7,0 g Calciumcarbonat versetzt. Anschließend läßt man unter Rühren bei 80 Grad Celsius 10,6 g Chlorameisensäurechlorethylester zutropfen. Die Lösung wird 3 Stunden lang bei dieser Temperatur gerührt. Sodann wird vom Rückstand abgesaugt und das Filtrat im Vakuum eingeengt. Der Rückstand wird an Kiesel gel chromatographiert (Laufmittel: Methylenchlorid mit 5 Prozent Methanol) und liefert nach der Kristallisation aus Wasser 3,4 g farblose Kristalle.
¹H-NMR-Spektrum (in CDCl₃):
8,35 (d, J₁ = 3 Hz, 6-H), 7,87 (dd, J₁ = 3 Hz, J₂ = 9 Hz, 4-H), 7,53 (d, J = 9 Hz, 3-H), 6,95 (breit, NH, beim Schütteln der Probe mit D₂O verschwindet das Signal, 4,58-4,32 (m, O-CH₂); 3,98 (q, J = 7 Hz, N-CH₂), 3,84-3,60 (m, Cl-CH₂), 1,21 (t, J = 7 Hz, CH₃).
Massenspektrum:
353 (3), 351 (18), 349 (28; m/e 353, 351, 349: M⁺), 314 (10), 244 (32), 243 (17), 242 (100), 241 (11), 206 (18), 152 (50), 93 (10), 79 (16), 55 (19), 53 (65).

### Stufe 4: 2,5-Bis(2'-chlorethoxycarbonylamino)-2-N-ethyl-6-nitro-pyridin

Die Nitrierung von 2,5 g der Verbindung der Stufe 3 mit 2,5 ml konzentrierter Salpetersäure in 10 ml konzentrierter Schwefelsäure analog Beispiel 1, Stufe 2 ergibt 2 g der Nitroverbindung vom Schmelzpunkt 87 Grad Celsius.
¹H-NMR-Spektrum (in CDCl₃):
9,55 (breit, NH), 8,94 (d, J = 9 Hz, 4-H), 8,21 (d, J = 9 Hz, 3-H), 4,48 (2x t, J = 6 Hz, OCH₂), 4,11 (q, J = 7 Hz, N-CH₂), 3,76 (2x t, J = 6 Hz, CH₂Cl), 1,31 (t, J = Hz, CH₃).
Massenspektrum
398 (2), 396 (10), 394 (15; m/e 398, 396, 394: M⁺), 377 (9), 240 (10), 178 (22), 80 (35), 65 (30), 63 (100).

### Beispiel 9: 2-Ethylamino-5-((2'-hydroxyethyl)amino)-6-nitro-pyridin

1,3 g des Bis-Carbamats des Beispiels 8, Stufe 4 werden in 20 ml 5-prozentiger Kaliumhydroxidlösung aufgeschlämmt und 2 1/2 Tage lang bei Raumtemperatur gerührt. Anschließend wird die Reaktionsmischung mit Eisessig auf einen pH-Wert von 3 bis 4 eingestellt und wiederholt mit Essigester extrahiert. Die vereinigten Extrakte werden über Natriumsulfat getrocknet und im Vakuum eingeengt. Nach Kristallisation aus Ethanol erhält man 0,23 g der Verbindung in Form grüner Kristalle.
¹H-NMR-Spektrum:
7,94 (t, J = 5 Hz, OH oder NH, Signal verschwindet beim Schütteln der Probe mit D₂O), 7,50 (d, J = 10 Hz, 4-H), 6,94 (d, J = 10 Hz, 3-H), 6,48 (t, J = 5 Hz, OH oder NH, Signal verschwindet beim Schütteln der Probe mit D₂O), 4,88 (t, J = 5 Hz, OH oder NH, Signal verschwindet beim Schütteln der Probe mit D₂O), 3,58 (t, J = 5 Hz, CH₂0H), 3,49-3,05 (m, N-CH₂), 1,14 (t, J = 7 Hz, CH₃).

### Beispiel 10: 5-(2'-Hydroxyethoxycarbonylamino)-2-methylamino-6-nitro-pyridin

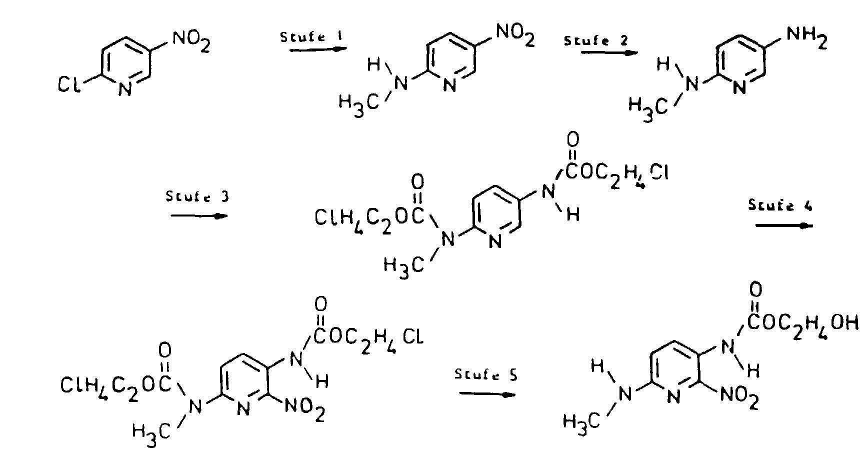

Wird die gesamte Reaktionssequenz analog Beispiel 8 (Methylaminlösung 40-prozentig an Stelle von Ethylamin) und Beispiel 9 durchgeführt, so erhält man 5-((2'-Hydroxyethyl)amino)-2-methylamino-6-nitro-pyridin in Form grüner Kristalle.

| Elementaranalyse: | | |
|---|---|---|
| | berechnet | gefunden |
| % C | 45,28 | 45,18 |
| % H | 5,69 | 5,40 |
| % N | 26,40 | 26,15 |

### Beispiel 11: 2,5-Bis((2'-hydroxyethyl)amino)-2-N-ethyl-6-nitro-pyridin

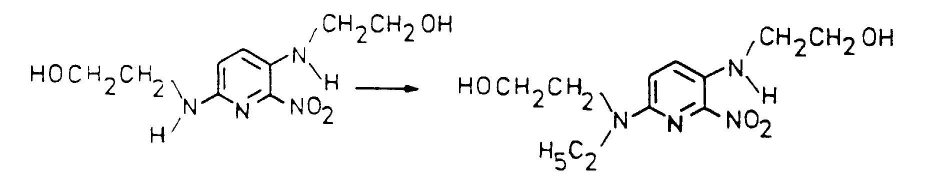

2,0 g der Bis(hydroxyethylamino)-Verbindung des Beispiels 7 werden in 50 ml Dioxan gelöst und mit 5 ml Ethyljodid versetzt. Nach Zugabe von 5 ml Triethanolamin wird 24 Stunden lang bei Raumtemperatur gerührt. Der Ansatz wird dann mit Wasser verdünnt, mit Essigsäure auf einen pH-Wert von 5 eingestellt und mit Essigester extrahiert. Die vereinigten Extrakte werden über Natriumsulfat getrocknet. Anschließend wird im Vakuum das Lösungsmittel verdampft und der Rückstand an Kieselgel (Laufmittel: Methylenchlorid mit 10 Prozent Methanol) chromatographiert.

Man erhält in geringer Ausbeute grüne Kristalle der gesuchten Verbindung.
¹H-NMR-Spektrum:
8,05 (t, J = 5 Hz, OH oder NH, Signal verschwindet beim Schütteln der Probe mit D₂O), 7,48 (d, J = 9 Hz, 4-H), 6,90 (d, J = 9 Hz, 3-H), 6,58 (t, J = 5 Hz, OH oder NH, Signal verschwindet beim Schütteln der Probe mit D₂O), 5,90 (breit, NH oder OH, Signal verschwindet beim Schütteln der Probe mit D₂O), 3,7-3,1 (m,-CH₂-OH und -NHCH₂-), 1,12 (t, J = 7 Hz, CH₃).

Die in dieser Anmeldung verwendeten Prozentzahlen stellen, soweit nicht anders angegeben, Gewichtsprozente dar.

## Patentansprüche

1. Verfahren zur Herstellung von 2,5-Diamino-6-nitro-pyiridinderivaten der allgemeinen Formel (I) 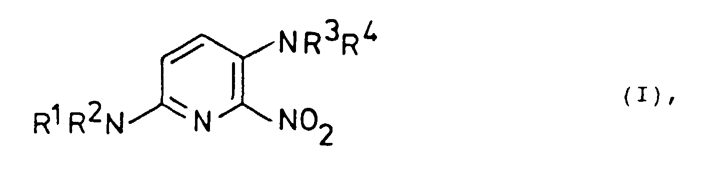 worin R¹ bis R⁴ unabhängig voneinander Wasserstoff oder einen der Reste Alkyl, Hydroxyalkyl, Dihydroxyalkyl, Alkoxyalkyl, Alkoxycarbonyl, hydroxy- oder chlorsubstituiertes Alkoxycarbonyl, Aminoalkyl sowie mit Alkyl- oder Hydroxylgruppen substituiertes Aminoalkyl bedeuten, wobei die Alkylreste jeweils 1 bis 4 Kohlenstoffatome aufweisen, oder durch R¹ und R² beziehungsweise R³ und R⁴ ein Oxazolidinonring gebildet wird, dadurch gekennzeichnet, daß ein 2,5-Diamino-pyridinderivat der allgemeinen Formel (II) 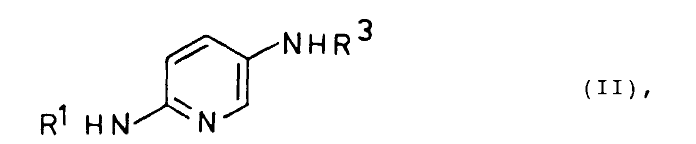 worin R¹ und R³ die vorstehend angegebene Bedeutung haben, mit einem Chlorameisensäurealkylester zu dem N²,N⁵-Bis-alkoxycarbonyl-2,5-diamino-pyridinderivat der allgemeinen Formel (III) 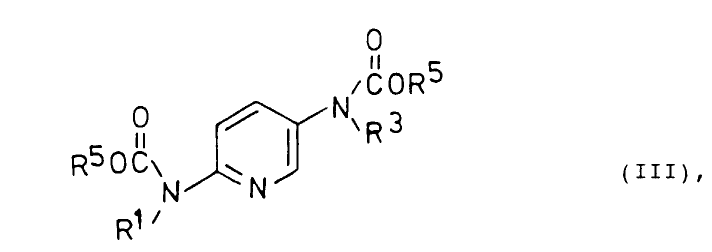 worin R¹ und R³ die vorstehend angegebene Bedeutung haben und R⁵ einen Alkyl-oder Chloralkylrest mit jeweils 1 bis 4 Kohlenstoffatomen darstellt, umgesetzt wird, dieses sodann in 6-Stellung zu dem N²,N⁵-Bis-alkoxycarbonyl-2,5-diamino-6-nitro-pyridinderivat der allgemeinen Formel (IV) 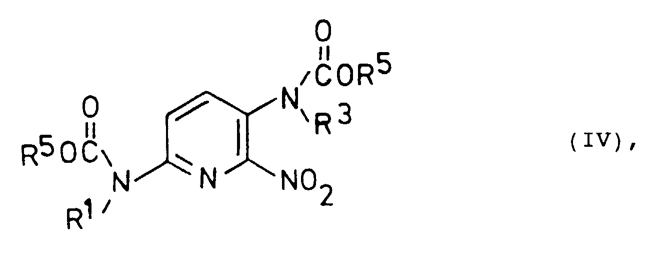 worin R¹, R³ und R⁵ die vorstehend angegebene Bedeutung haben, nitriert wird und anschließend, falls die Verbindung der Formel (I) keine Alkoxycarbonylreste aufweist, das Produkt (IV) durch Abspaltung der Alkoxycarbonylreste und durch darauffolgende, für die Alkylierung von aromatischen Aminen bekannte Alkylierungsreaktionen mit Alkylierungsmitteln, durch welche die vorstehend für R¹ bis R⁴ angegebenen Reste eingeführt werden können, in das 2,5-Diamino-pyridinderivat der allgemeinen Formel (I) übergeführt wird.

2. Verfahren nach Anspruch 1, dadurch gekennzeichnet, daß die Reste R¹ bis R⁴ unabhängig voneinander Wasserstoff, Methyl, Ethyl oder 2-Hydroxyethyl bedeuten.

3. Verfahren nach Anspruch 1, dadurch gekennzeichnet, daß durch die Reste R¹ und R² und/oder R³ und R⁴ ein Oxazolidinonring gebildet wird.

4. Verfahren nach Anspruch 1, dadurch gekennzeichnet, daß R¹ die in Anspruch 1 angegebene Bedeutung hat, jedoch nicht Wasserstoff ist, und die Reste R², R³ und R⁴ Wasserstoff darstellen.

5. Verfahren nach Anspruch 1, dadurch gekennzeichnet, daß die Reste R¹ bis R⁴ Wasserstoff bedeuten.

6. Verfahren nach Anspruch 1, dadurch gekennzeichnet, daß 2,5-Bis((2'-hydroxyethyl)amino)-6-nitro-pyridin hergestellt wird indem man 2,5-Diamino-pyridin mit Chlorameisensäurechlorethylester zum N²,N⁵-Bis(2'-chlorethoxycarbonyl)-2,5-diamino-pyridin umsetzt, dieses sodann in 6-Stellung nitriert und anschließend die 2-Chlorethoxycarbonylreste durch Umsetzung mit Ammoniak oder Alkylaminen in Oxazolidinonreste umwandelt, welche danach durch Einwirkung von ethanolischer Alkalihydroxidlösung zu 2-Hydroxyethylresten umgesetzt werden.

7. 2,5-Diamino-6-nitro-pyridinderivat der allgemeinen Formel (V) 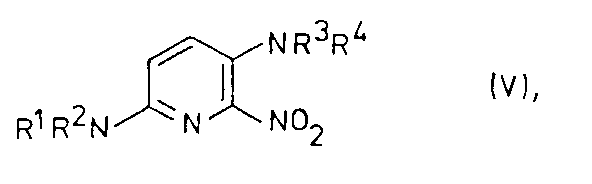 in der R¹ bis R⁴ die in Anspruch 1 angegebene Bedeutung haben, mit der Maßgabe, daß R¹ bis R⁴ nicht ausschließlich Wasserstoff bedeuten und daß, wenn R¹ und R² Alkyl bedeuten, R³ und R⁴ von Wasserstoff verschieden sind.

8. 2,5-Bis((2'-hydroxyethyl)amino)-6-nitro-pyridin.

9. 2-Ethylamino-5-((2'-hydroxyethyl)amino)-6-nitro-pyridin.

10. 2,5-Bis((2'-hydroxyethyl)amino)-2-N-ethyl-6-nitro-pyridin.

11. 5-Amino-2-((2'-hydroxyethyl)amino)-6-nitro-pyridin.

12. 2,5-Bis(1',3'-oxazolidin-3'-yl-2'-on)-6-nitro-pyridin.

## Claims

1. Process for producing 2,5-diamino-6-nitro-pyridine derivatives of the general formula (I) 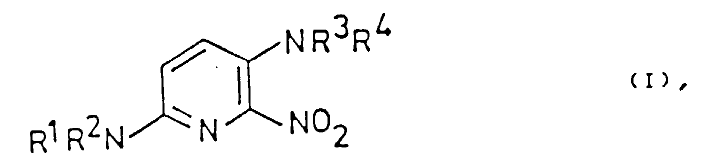 wherein R¹ to R⁴ independently from each other mean hydrogen or one of the residues alkyl, hydroxyalkyl,dihydroxyalkyl, alkoxyalkyl, alkoxycarbonyl,hydroxy- or chlorine-substituted alkoxycarbonyl, aminoalkyl as well as aminoalkyl substituted with alkyl- or hydroxy-groups wherein the alkyl residues each have 1 to 4 carbon atoms, or an oxazolidinone ring is formed by R¹ and/or R² or R³ and R⁴
characterised in that a 2,5-diamino-pyridine derivative of the general formula (II) 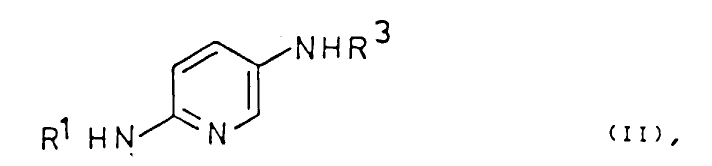 wherein R¹ and R³ have the meaning given above is converted with a chloroformic acid alkyl ester to the N², N⁵, bis alkoxycarbonyl-2,5 diamino pyridine derivative of the general formula (111) 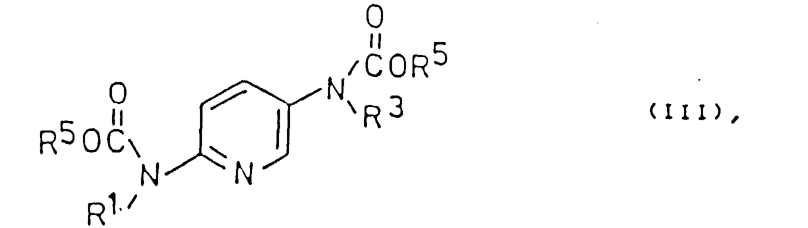 wherein R¹ and R³ have the meaning given above and R⁵ represents an alkyl or chloroalkyl residue with in each case 1 to 4 carbon atoms, this is then nitrated in the 6 position to the N²,N⁵-bis-alkoxycarbonyl-2,5-diamino-6-nitro-pyridine derivative of the general formula (IV) 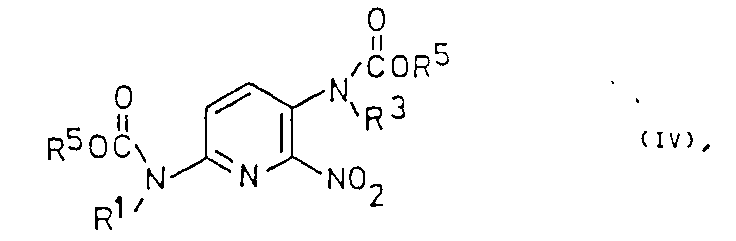 wherein R¹, R³ and R⁵ have the meaning given above, and, finally, if the compound of the formula (I) has no alkoxycarbonyl residue, the product (IV) is converted by cleavage of the alkoxycarbonyl residue and by a following alkylation reaction with an alkylation medium known for alkylating aromatic amines, by means of which the residues given above for R¹ to R⁴ can be introduced, into the 2,5-diamino-pyridine derivative of the general formula (I).

2. Process according to Claim 1, characterised in that the residues R¹ to R⁴ mean hydrogen, methyl, ethyl or 2-hydroxyethyl, independently of each other.

3. Process according to Claim 1, characterised in that an oxazolidinone ring is formed by the residues R¹ and R² or R³ and R⁴.

4. Process according to Claim 1, characterised in that R¹ has the meaning given in Claim 1, but is not hydrogen and the residues R², R³ and R⁴ represent hydrogen.

5. Process according to Claim 1, characterised in that the residues R¹ to R⁴ mean hydrogen.

6. Process according to Claim 1, characterised in that 2,5 - bis ((2'-hydroxyethyl)amino)- 6 nitropyridine is produced, by reacting 2,5 diamino-pyridine with chloroformic acid chloroethyl ester to N²,N⁵-bis-(2'chlorethoxycarbonyl) 2,5-diamino-pyridine, this is then nitrated in the 6-position and finally the 2-chlorethoxycarbonyl residues are converted by reaction with ammonia or alkylamines to oxazolidinone residues, which are then converted by the action of an ethanolic alkali metal hydroxide solution into 2-hydroxyethyl residues.

7. 2,5 - diamino-6-nitro-pyridine derivative of the general formula (V) 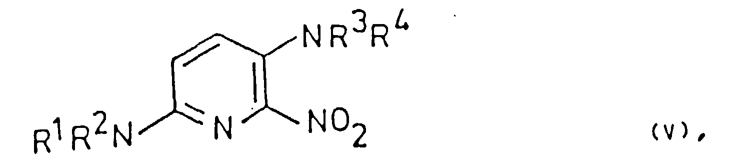 in which R¹ to R⁴ have the meaning given in Claim 1, with the proviso that R¹ to R⁴ do not only mean hydrogen and that if R¹ and R² mean alkyl, R³ and R⁴ are different from hydrogen.

8. 2,5-bis(2'-hydroxyethyl)amino)-6-nitro-pyridine.

9. 2-ethyl amino-5-((2'-hydroxyethyl)amino)-6-nitro-pyridine.

10. 2,5-bis((2'-hydroxyethyl)amino)-2-N-ethyl-6-nitro-pyridine.

11. 5-amino-2-((2'-hydroxyethyl)amino)-6-nitro-pyridine.

12. 2,5 - bis (1',3-oxazolidine-3'-yl-2'-one) 6 -nitro-pyridine.

## Revendications

1. Procédé de préparation de dérivés de la 2,5-diamino-6-nitro-pyridine répondant à la formule générale (I) 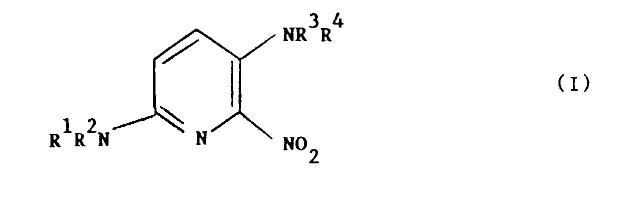 dans laquelle R¹ à R⁴ représentent, indépendamment les uns des autres, l'hydrogène ou l'un des restes alkyle, hydroxyalkyle, dihydroxyalkyle, alkoxyalkyle, alkoxycarbonyle, alkoxycarbonyle hydroxy- ou chloro-substitué, amino-alkyle, et amino-alkyle substitué par des groupes alkyle ou hydroxyle, les restes alkyle comportant chacun 1 à 4 atomes de carbone, ou bien un noyau oxazolidinonique étant formé par R¹ et R², ou bien R³ et R⁴, caractérisé en ce qu'on fait réagir un dérivé de la 2, 5-diamino-pyridine répondant à la formule générale (II) 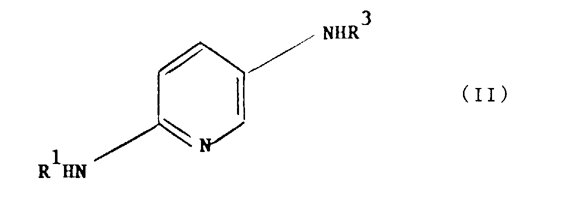 dans laquelle R¹ et R³ ont la signification indiquée précédemment, sur un chloroformiate d'alkyle pour aboutir au dérivé N², N⁵-bis-alkoxycarbonyl-2,5-diamino-pyridinique répondant à la formule (III) 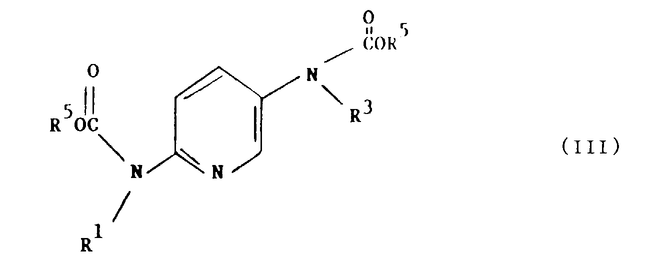 dans laquelle R¹ et R³ ont la signification indiquée précédemment et R⁵ représente un reste alkyle ou chloro-alkyle comportant chacun 1 à 4 atomes de carbone, ensuite on le nitre en position 6 pour aboutir au dérivé N², N⁵-bis-alkoxycarbonyl-2,5-diamino-6-nitro-pyridinique répondant à la formule générale (IV) 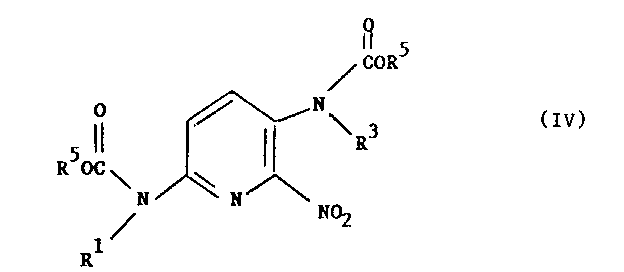 dans laquelle R¹, R³ et R⁵ ont la signification indiquée précédemment, et ensuite dans le cas où le composé de la formule (I) ne comporte pas de restes alkoxycarbonyle, on transforme le produit (IV) en le dérivé de 2,5-diamino-pyridine répondant à la formule générale (I) par séparation des restes alkoxycarbonyle et par des réactions d'alkylation connues pour l'alkylation d'amines aromatiques qui suivent, avec des agents d'alkylation qui peuvent introduire les restes indiqués précédemment pour R¹ à R⁴.

2. Procédé selon la revendication 1, caractérisé en ce que les restes R¹ à R⁴ représentent, indépendamment les uns des autres, l'hydrogène ou bien un groupe méthyle, éthyle ou 2-hydroxyéthyle.

3. Procédé selon la revendication 1, caractérisé en ce que les restes R¹ et R² et/ou R³ et R⁴ forment un noyau oxazolidinonique.

4. Procédé selon la revendication 1, caractérisé en ce que R¹ a la signification indiquée dans la revendication 1, mais n'est pas l'hydrogène, et les restes R², R³ et R⁴ représentent l'hydrogène.

5. Procédé selon la revendication 1, caractérisé en ce que les restes R¹ à R⁴ représentent l'hydrogène.

6. Procédé selon la revendication 1, caractérisé en ce qu'on prépare la 2,5-bis((2'-hydroxyéthyl)amino)-6-nitropyridine en faisant réagir la 2,5-diamino-pyridine sur du chloroformiate de chloro-éthyle pour aboutir à la N²,N⁵-bis-(2'-chloroéthoxycarbonyl)-2,5-diamino-pyridine, puis on la nitre en position 6, et l'on transforme ensuite les restes 2-chloroéthoxycarbonyle par réaction avec de l'ammoniac ou des alkylamines en restes oxazolidinoniques que l'on transforme ensuite en reste 2-hydroxyéthyl par action d'une solution d'hydroxyde alcalin dans l'éthanol.

7. Dérivé de la 2,5-diamino-6-nitro-pyridine répondant à la formule générale (V) 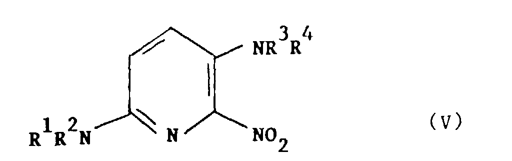 dans laquelle R¹ à R⁴ ont la signification indiquée dans la revendication 1, sous réserve que R¹ à R⁴ ne représentent pas exclusivement l'hydrogène et que, lorsque R¹ et R² représentent un alkyle, R³ et R⁴ sont différents de l'hydrogène.

8. 2,5-bis ((2'-hydroxyéthyl)amino)-6-nitro-pyridine.

9. 2-éthylamino-5-((2'-hydroxyéthyl)amino)-6-nitro-pyridine.

10. 2,5-bis ((2'-hydroxyéthyl)amino)-2-N-éthyl-6-nitro-pyridine.

11. 5-amino-2-((2'-hydroxyéthyl)amino)-6-nitro-pyridine.

12. 2,5-bis (1', 3'-oxazolidin-3'-yl-2'-on)-6-nitro-pyridine.
